# EUROPÄISCHE PATENTSCHRIFT

(11) **EP 3 558 153 B1**
(45) Veröffentlichungstag und Bekanntmachung des Hinweises auf die Patenterteilung: **29.10.2025**
(21) Anmeldenummer: 17825461.1
(22) Anmeldetag: 15.12.2017
(51) Int. Cl.: A61B 90/70, A61B 90/90, A61B 90/98, A61B 90/96

(54) **VERFAHREN ZUM BETREIBEN EINER AUFBEREITUNGSVORRICHTUNG SOWIE EINES MEDIZINISCHEN SYSTEMS**
METHOD FOR OPERATING A RECONDITIONING APPARATUS AND A MEDICAL SYSTEM
PROCÉDÉ POUR FAIRE FONCTIONNER UN DISPOSITIF DE PRÉPARATION ET UN SYSTÈME MÉDICAL

(30) Priorität: 21.12.2016 DE 102016225884
(43) Veröffentlichungstag der Anmeldung: 30.10.2019
(73) Patentinhaber: Olympus Winter & Ibe GmbH, 22045 Hamburg (DE)
(72) Erfinder: WEIS, Antonia, 22047 Hamburg (DE)
(74) Vertreter: Seemann & Partner Patentanwälte mbB
(86) Internationale Anmeldenummer: PCT/EP2017/082959
(87) Internationale Veröffentlichungsnummer: WO 2018/114639

(56) Entgegenhaltungen:
- EP-A1- 2 740 401
- US-A1- 2009 220 377

## Beschreibung

### Beschreibung

Die Erfindung betrifft ein Verfahren zum Betreiben einer Aufbereitungsvorrichtung zum Reinigen und Desinfizieren zumindest eines medizinischen Instruments, welches zumindest einen innenliegenden Kanal aufweist, wobei das medizinische Instrument während der Aufbereitung in einem Aufbereitungsraum der Aufbereitungsvorrichtung aufgenommen und der zumindest eine innenliegende Kanal mit einem in dem Aufbereitungsraum vorhandenen Fluidanschluss einer Mehrzahl vorhandener Fluidanschlüsse verbunden wird, wobei der Kanal während der Aufbereitung mit einem Aufbereitungsfluid gespült wird. Die Erfindung betrifft ebenso ein Verfahren zum Betreiben eines medizinischen Systems, umfassend eine Aufbereitungsvorrichtung zum Aufbereiten zumindest eines medizinischen Instruments und ein medizinisches Instrument mit zumindest einem innenliegenden Kanal. Ferner betrifft die Erfindung eine Aufbereitungsvorrichtung zum Reinigen und Desinfizieren zumindest eines medizinischen Instruments mit zumindest einem innenliegenden Kanal, wobei die Aufbereitungsvorrichtung einen Aufbereitungsraum umfasst, der zur Aufnahme des medizinischen Instruments während der Aufbereitung eingerichtet ist, und wobei der zumindest eine innenliegende Kanal mit einem in dem Aufbereitungsraum vorhandenen Fluidanschluss einer Mehrzahl vorhandener Fluidanschlüsse zum Spülen des Kanals mit einem Aufbereitungsfluid verbindbar oder verbunden ist. Ebenso betrifft die Erfindung ein medizinisches System, umfassend eine Aufbereitungsvorrichtung.

An die Aufbereitung von medizinischen Geräten und Instrumenten, beispielsweise von medizinischen Instrumenten wie Endoskopen, werden hohe Anforderungen gestellt. Nach der Benutzung der medizinischen Instrumente werden diese in einem Reinigungs-und/oder Desinfektionsgerät, welches allgemein als Aufbereitungsvorrichtung bezeichnet werden soll, desinfiziert und/oder gereinigt. Während der Aufbereitung befindet sich das medizinische Instrument in einem Aufbereitungsraum der Aufbereitungsvorrichtung. Eine solche Aufbereitungsvorrichtung für Endoskope ist beispielsweise unter der Bezeichnung ETD (Endo Thermodesinfektor) des Herstellers OLYMPUS Winter & Ibe GmbH, Hamburg, bekannt.

Innenliegende Kanäle medizinischer Instrumente, beispielsweise innenliegende Arbeitskanäle von Endoskopen, werden bei der Aufbereitung vielfach durch Spülen mit einer Reinigungs- und/oder Desinfizierlösung gereinigt. Eine Reinigungs- und/oder Desinfizierlösung soll allgemein als Aufbereitungsfluid bezeichnet werden. Um das Aufbereitungsfluid durch die innenliegenden Kanäle des medizinischen Instruments zu leiten, wird dieses mit einem oder mehreren Fluidanschlüssen verbunden, die in dem Aufbereitungsraum vorhanden sind.

Die Aufbereitung der medizinischen Instrumente findet vielfach in mehreren aufeinanderfolgenden Spülschritten statt, in denen die innenliegenden Kanäle mit verschiedenen Aufbereitungsfluiden gespült werden. Zur Minimierung der Laugenverschleppung, also der Verschleppung der nach dem Abpumpen in den Kanälen verbleibenden Restmenge an Aufbereitungsfluid eines ersten Spülschrittes in einen darauffolgenden zweiten Spülschritt, gibt es verschiedene Ansätze. Neben einer fallenden Verschlauchung ist es beispielsweise möglich, Druckluft einzusetzen, um Restmengen an Aufbereitungsfluid zu beseitigen.

Aus der EP 2 740 401 A1 ist ein Aufbereitungsgerät für Endoskope bekannt, welches verschiedene Anschlüsse umfasst, die mit zu spülenden Kanälen des Endoskops verbunden werden. Ein Endoskoptyp wird erkannt und es wird ein individuelles Aufbereitungsprogramm für den erkannten Endoskoptyp durchgeführt. Um eine korrekte Zuordnung der Anschlüsse des Aufbereitungsgeräts mit den Anschlüssen des Endoskops zu erleichtern, stellt das Aufbereitungsgerät einem Benutzer Informationen auf einer Anzeige zur Verfügung. Die korrekte Zuordnung kann ferner durch Leuchtdioden unterstützt werden, die sich neben den Anschlüssen des Aufbereitungsgeräts befinden. Um anschließend die korrekte Belegung der Anschlüsse zu überprüfen, kann eine Druckmessung vorgenommen werden, welche anhand von Druckwerten oder eines Druckabfalls die korrekte Belegung der Anschlüsse überprüft.

Aus US 2009/220377 A1 ist eine Wasch- und Desinfektionsvorrichtung für Endoskope bekannt, die eine Mehrzahl von Verbindungsanschlüssen umfasst, die ebenfalls mit entsprechenden Anschlüssen des Endoskops verbunden werden. Das Endoskop umfasst einen RFID-Tag, auf dem Identifikationsinformationen, die für das Endoskop spezifisch sind, abgelegt sind. Eine Kontrolleinheit überwacht eine Flussrate der Spüllösungen in den Kanälen des Endoskops und prüft, ob diese Werte im Hinblick auf die Identifikationsinformationen des Endoskops korrekt sind.

Aus der DE 10 2014 102 064 A1 ist eine Vorrichtung zum Reinigen von Medizinprodukten bekannt, bei der Druckluft eingesetzt wird. Diese umfasst eine Kammer zur Aufnahme der zu reinigenden Gegenstände und zumindest eine Zuleitung zur Zuführung eines Reinigungsfluids. Zur Verteilung des Reinigungsfluids auf oder in die zu reinigenden Gegenstände ist eine entsprechende Vorrichtung vorgesehen. In die Zuleitung für das Reinigungsfluid kann mittels eines Ventils Druckluft eingespeist werden. Als Reinigungsfluid wird eine Mischung aus einer wässrigen Reinigungsflüssigkeit und Druckluft bereitgestellt.

Druckluft ist ein wertvolles und teures Arbeitsfluid, dessen Herstellungs- und Bereitstellungskosten in vielen Fällen unterschätzt werden. Ein ökonomischer Umgang mit dieser Ressource ist also stets erstrebenswert.

Es ist eine Aufgabe der Erfindung, ein Verfahren zum Betreiben einer Aufbereitungsvorrichtung, ein Verfahren zum Betreiben eines medizinischen Systems, eine Aufbereitungsvorrichtung zum Reinigen und Desinfizieren zumindest eines medizinischen Instruments sowie ein medizinisches System anzugeben, wobei ein wirtschaftlicher Umgang mit Druckluft als Prozessfluid angestrebt wird. In diesem Zusammenhang sollen ferner auch die baulichen Grenzen der Ressource Druckluft, beispielsweise im Hinblick auf den verfügbaren Volumenstrom der Druckluft, beachtet werden.

Die Aufgabe wird gelöst durch ein Verfahren nach Anspruch 1 und eine Aufbereitungsanlage nach Anspruch 6. Weitere Ausführungsformen werden in den abhängigen Ansprüchen offenbart. Beschrieben wird ein Verfahren zum Betreiben einer Aufbereitungsvorrichtung zum Reinigen und Desinfizieren zumindest eines medizinischen Instruments, welches zumindest einen innenliegenden Kanal aufweist, wobei das medizinische Instrument während der Aufbereitung in einem Aufbereitungsraum der Aufbereitungsvorrichtung aufgenommen und der zumindest eine innenliegende Kanal mit einem in dem Aufbereitungsraum vorhandenen Fluidanschluss einer Mehrzahl vorhandener Fluidanschlüsse verbunden wird, wobei der Kanal während der Aufbereitung mit einem Aufbereitungsfluid gespült wird, wobei das Verfahren dadurch fortgebildet ist, dass die Aufbereitungsvorrichtung eine Steuereinheit, eine Drucklufteinheit, eine Datenbank und eine Eingabeeinheit zum Erfassen eines Kennzeichnungsmerkmals umfasst, wobei in der Datenbank eine Mehrzahl von Datensätzen gespeichert ist, die jeweils Informationen betreffend eine Belegung der in dem Aufbereitungsraum vorhandenen Fluidanschlüsse durch einen bestimmten Typ eines medizinischen Instruments umfassen, wobei mit der Eingabeeinheit ein Kennzeichnungsmerkmal des medizinischen Instruments erfasst wird, auf dem Informationen betreffend seinen Typ gespeichert sind, die Steuereinheit den anhand der erfassten Information identifizierten Typ des medizinischen Instruments mit in der Datenbank vorhandenen Datensätzen abgleicht und so eine Belegung des mit dem innenliegenden Kanal verbundenen Fluidanschlusses anhand des identifizierten Typs feststellt und die Drucklufteinheit derart ansteuert, dass diese den belegten Fluidanschluss mit einem Druckluftstoß beaufschlagt, so dass in dem gespülten innenliegenden Kanal des medizinischen Instruments vorhandenes Aufbereitungsfluid aus dem Kanal ausgeblasen wird, wobei die Aufbereitung des medizinischen Instruments mehrere Spülschritte umfasst, und wobei zwischen zwei aufeinanderfolgenden Spülschritten der zumindest eine belegte Fluidanschluss mit dem Druckluftstoß beaufschlagt wird.

Vorteilhaft wird bei dem Verfahren zum Betreiben der Aufbereitungsvorrichtung gemäß Aspekten der Erfindung lediglich so viel Druckluft verwendet, wie zum Erzielen des gewünschten technischen Effekts auch tatsächlich notwendig ist. Mit anderen Worten wird also keine Druckluft in irgendeiner Weise verschwendet. Ebenfalls sinken vorteilhaft die Anforderungen im Hinblick auf den seitens der Infrastruktur bereitzustellenden Volumenstrom der Druckluft. Beispielsweise muss das Druckluftsystem nicht mehr auf selten auftretende Spitzenluftströme ausgelegt werden, da diese nicht mehr oder in vermindertem Maße auftreten. Ein hoher Druckluftverbrauch führt zu hohen Betriebskosten. Diese sinken bei dem Verfahren zum Betreiben der Aufbereitungsvorrichtung vorteilhaft. Die Druckluftbeaufschlagung der Fluidanschlüsse erfolgt in Kenntnis einer Belegung dieser Anschlüsse durch das angeschlossene Spülgut, also des medizinischen Instruments. Es erfolgt eine spezifische Druckluftbeaufschlagung lediglich derjenigen Anschlüsse, die auch tatsächlich während der Aufbereitung mit Aufbereitungsfluid gespült werden und daher zur Verringerung der Laugenverschleppung ausgeblasen werden.

Im Aufbereitungsraum ist eine Anzahl von Fluidanschlüssen vorhanden. Durch die individuelle Erfassung des Typs des aufbereiteten medizinischen Instruments werden lediglich diejenigen Fluidanschlüsse, also diejenige Teilmenge der vorhandenen Fluidanschlüsse, mit dem Druckluftstoß beaufschlagt, die von dem entsprechenden Typ des medizinischen Instruments auch belegt werden. Es ist insbesondere nicht vorgesehen, dass einfach alle Fluidanschlüsse mit dem Druckluftstoß beaufschlagt werden. Bei diesem Vorgehen würde ein Teil der eingesetzten Druckluft über die nicht belegten Fluidanschlüsse ungenutzt in den Aufbereitungsraum entweichen. Dies wäre eine Verschwendung von Druckluft, was die Betriebskosten der Aufbereitungsvorrichtung unnötig und nutzlos erhöhen würde.

Die Erfassung des Kennzeichnungsmerkmals erfolgt beispielsweise durch eine manuelle Eingabe. In einem solchen Fall ist die Eingabeeinheit beispielsweise eine Tastatur, ein Ziffernblock oder dergleichen.

Erfindungsgemäß ist vorgesehen, dass die Aufbereitung des medizinischen Instruments mehrere Spülschritte umfasst, wobei zwischen zwei aufeinanderfolgenden Spülschritten der zumindest eine belegte Fluidanschluss mit dem Druckluftstoß beaufschlagt wird. Vorteilhaft wird die Vermischung der Aufbereitungsfluide, wie sie in den aufeinanderfolgenden Spülschritten eingesetzt werden, stark verringert.

Ferner ist insbesondere vorgesehen, dass von dem identifizierten Typ von medizinischem Instrument nicht belegte Fluidanschlüsse zumindest während der Dauer des Druckluftstoßes abgesperrt werden, so dass aus den nicht belegten Fluidanschlüssen keine Druckluft entweicht. Vorteilhaft wird so sichergestellt, dass während des Druckluftstoßes tatsächlich auch nur diejenigen Fluidanschlüsse beaufschlagt werden, welche mit einem innenliegenden Kanal des medizinischen Instruments fluidisch verbunden sind.

Ferner ist insbesondere vorgesehen, dass nach Beendigung des Reinigungs- und Desinfektionsvorgangs, also nach Beendigung des Aufbereitungsvorgangs, eine Beaufschlagung der angeschlossenen Fluidanschlüsse mit dem Druckluftstoß erfolgt. Eine solche Maßnahme kann beispielsweise vorgesehen sein, um die innenliegenden Kanäle zu trocknen.

Gemäß einer weiteren Ausführungsform ist das Verfahren dadurch fortgebildet, dass von dem identifizierten Typ von medizinischem Instrument nicht belegte Fluidanschlüsse mit einem weiteren Druckluftstoß beaufschlagt werden, wobei dieser weitere Druckluftstoß insbesondere zeitlich kürzer ist als der Druckluftstoß mit dem der zumindest eine gespülte innenliegende Kanal des medizinischen Instruments ausgeblasen wird. Der Vergleich zwischen dem Druckluftstoß und dem weiteren Druckluftstoß im Hinblick auf die zeitliche Dauer der beiden Luftdruckstöße erfolgt insbesondere und sinnvollerweise bei gleichem Druck. Mit anderen Worten hat also der weitere Druckluftstoß eine geringere Masse als der Druckluftstoß. Der weitere Druckluftstoß dient dazu Reinigungsfluid, welches sich während des Aufbereitungsvorgangs in den nicht belegten Fluidanschlüssen sammelt, auszublasen, sodass am Ende des Aufbereitungsvorgangs das System so sauber wie möglich ist. Für den weiteren Druckluftstoß ist eine kürzere zeitliche Dauer ausreichend, da in den nicht belegten Fluidanschlüssen potenziell weniger Flüssigkeit vorhanden ist als in den belegten Anschlüssen.

Das Verfahren ist ferner vorteilhaft dadurch weitergebildet, dass das Kennzeichnungsmerkmal des medizinischen Instruments ein maschinenlesbares Kennzeichnungsmerkmal ist und die Eingabeeinheit ein Lesegerät zum Erfassen des maschinenlesbaren Kennzeichnungsmerkmals ist, wobei das Kennzeichnungsmerkmal erfasst wird, indem dieses von dem Lesegerät ausgelesen wird. Ein maschinenlesbares Kennzeichnungsmerkmal ist beispielsweise ein RFID-Tag, ein Barcode oder auch ein QR-Code.

Im Kontext der vorliegenden Beschreibung ist ein "Fluidanschluss" ein Anschluss für beispielsweise eine mit einem Schlauch verbundene Kupplung an dem ein Fluid, also eine Flüssigkeit, ein Gas oder ein Flüssigkeits-Gas-Gemisch, bereitgestellt werden. Ein Fluidanschluss ist beispielsweise ein Anschluss an den eine Schnellkupplung eines Schlauchs ankoppelbar ist, durch den ein Aufbereitungsfluid geleitet wird, und der mit seinem gegenüberliegenden Ende mit einem Endoskop koppelbar ist. Unter dem Begriff "fluidisch gekoppelt" ist eine mittelbare oder unmittelbare Verbindung für ein Fluid zu verstehen. Beispielsweise eine Rohrleitung oder ein Schlauch für das Arbeitsfluid.

Die Aufgabe wird ferner gelöst durch ein Verfahren zum Betreiben eines medizinischen Systems, umfassend eine Aufbereitungsvorrichtung zum Aufbereiten zumindest eines medizinischen Instruments und ein medizinisches Instrument mit zumindest einen innenliegenden Kanal, wobei das Verfahren dadurch fortgebildet ist, dass das medizinische System nach einem Verfahren gemäß einem oder mehreren der zuvor genannten Aspekte betrieben wird.

Auf das Verfahren zum Betreiben des medizinischen Systems treffen gleiche oder ähnliche Vorteile zu, wie sie bereits im Hinblick auf das Verfahren zum Betreiben der Aufbereitungsvorrichtung erwähnt wurden.

Ferner wird die Aufgabe gelöst durch eine Aufbereitungsvorrichtung zum Reinigen und Desinfizieren zumindest eines medizinischen Instruments mit zumindest einem innenliegenden Kanal, wobei die Aufbereitungsvorrichtung einen Aufbereitungsraum umfasst, der zur Aufnahme des medizinischen Instruments während der Aufbereitung eingerichtet ist, und wobei der zumindest eine innenliegende Kanal mit einem in dem Aufbereitungsraum vorhandenen Fluidanschluss einer Mehrzahl vorhandener Fluidanschlüsse zum Spülen des Kanals mit einem Aufbereitungsfluid verbindbar oder verbunden ist, wobei die Aufbereitungsvorrichtung dadurch fortgebildet ist, dass die Aufbereitungsvorrichtung eine Steuereinheit, eine Drucklufteinheit, eine Datenbank und eine Eingabeeinheit zum Erfassen eines Kennzeichnungsmerkmals umfasst, wobei in der Datenbank eine Mehrzahl von Datensätzen gespeichert ist, die jeweils Informationen betreffend eine Belegung der in dem Aufbereitungsraum vorhandenen Fluidanschlüsse durch einen bestimmten Typ eines medizinischen Instruments umfassen, und wobei die Eingabeeinheit dazu eingerichtet ist, ein Kennzeichnungsmerkmal des medizinischen Instruments zu erfassen, in welchem Informationen betreffend den Typ des medizinischen Instruments gespeichert sind, wobei die Steuereinheit ferner dazu eingerichtet ist, den anhand der ausgelesenen Information identifizierten Typ des medizinischen Instruments mit in der Datenbank vorhandenen Datensätzen abzugleichen und so eine Belegung des mit dem innenliegenden Kanal verbundenen Fluidanschlusses anhand des identifizierten Typs festzustellen und ferner die Drucklufteinheit derart anzusteuern, dass diese den belegten Fluidanschluss mit einem Druckluftstoß beaufschlagt, so dass in dem gespülten innenliegenden Kanal des medizinischen Instruments vorhandenes Aufbereitungsfluid aus dem Kanal ausgeblasen werden kann, wobei die Aufbereitung des medizinischen Instruments mehrere Spülschritte umfasst, und wobei die Steuereinheit ferner dazu eingerichtet ist, zwischen zwei aufeinanderfolgenden Spülschritten den zumindest einen belegten Fluidanschluss mit dem Druckluftstoß zu beaufschlagen.

Auch auf die Aufbereitungsvorrichtung treffen gleiche oder ähnliche Vorteile zu, wie sie bereits im Hinblick auf das Verfahren zum Betreiben der Aufbereitungsvorrichtung erwähnt wurden. Vorteilhaft benötigt die Aufbereitungsvorrichtung nur eine sehr geringe Menge an Druckluft als Prozessfluid. Aus diesem Grund ist die Aufbereitungsvorrichtung sehr ökonomisch im Hinblick auf ihre Betriebskosten.

Gemäß einer weiteren Ausführungsform ist die Aufbereitungsvorrichtung dadurch fortgebildet, dass die Steuereinheit ferner dazu eingerichtet ist, von dem identifizierten Typ von medizinischem Instrument nicht belegte Fluidanschlüsse mit einem weiteren Druckluftstoß zu beaufschlagen, wobei dieser weitere Druckluftstoß insbesondere zeitlich kürzer ist als der Druckluftstoß mit dem der zumindest eine belegte Fluidanschluss beaufschlagt wird.

Die Steuereinheit ist also dazu eingerichtet zu erkennen, welche Fluidanschlüsse von dem medizinischen Instrument belegt werden welche nicht. Diese Information gewinnt die Steuereinheit aus der in dem Kennzeichnungsmerkmal gespeicherten Information.

In einer weiteren vorteilhaften Ausführungsform ist vorgesehen, dass die Aufbereitungsvorrichtung derart eingerichtet ist, dass das Kennzeichnungsmerkmal des medizinischen Instruments ein maschinenlesbares Kennzeichnungsmerkmal ist und die Eingabeeinheit ein Lesegerät zum Erfassen des maschinenlesbaren Kennzeichnungsmerkmals ist, wobei die Steuereinheit ferner dazu eingerichtet ist, das Kennzeichnungsmerkmal zu erfassen, indem dieses von dem Lesegerät ausgelesen wird. Die Aufbereitungsvorrichtung umfasst als Lesegerät beispielsweise einen RFID-Leser, einen Barcodescanner oder dergleichen.

Ferner wird die Aufgabe gelöst durch ein medizinisches System, umfassend eine Aufbereitungsvorrichtung gemäß einem oder mehreren der zuvor genannten Aspekte. Das medizinische System umfasst ferner zumindest ein medizinisches Instrument mit zumindest einem innenliegenden Kanal, wobei das medizinische Instrument ein maschinenlesbares Kennzeichnungsmerkmal umfasst, in dem Informationen betreffend den Typ des medizinischen Instruments gespeichert sind.

Das medizinische Gerät ist insbesondere ein chirurgisches Instrument, beispielsweise ein Endoskop. Dies betrifft vorteilhaft alle Ausführungsformen.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein medizinisches System, umfassend eine Aufbereitungsvorrichtung und ein medizinisches Instrument, in einer schematisch vereinfachten perspektivischen Darstellung und
- Fig. 2: eine schematisch vereinfachte Draufsicht auf einen Aufbereitungsraum einer Aufbereitungsvorrichtung, in dem ein medizinisches Instrument angeordnet ist.

In den Zeichnungen sind jeweils gleiche oder gleichartige Elemente und/oder Teile mit denselben Bezugsziffern versehen, so dass von einer erneuten Vorstellung jeweils abgesehen wird.

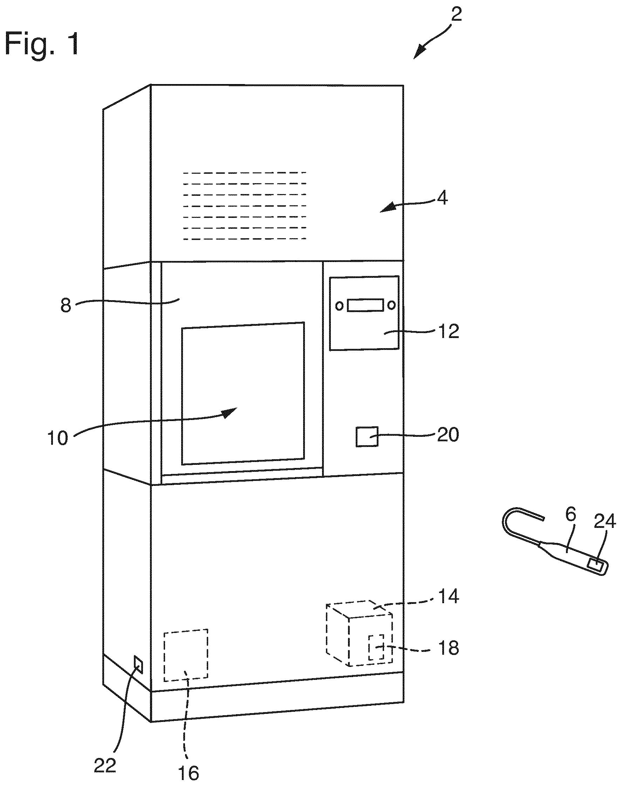

Fig. 1 zeigt in schematisch vereinfachter und perspektivischer Ansicht ein medizinisches System 2, umfassend eine Aufbereitungsvorrichtung 4 und ein medizinisches Instrument 6. Die Aufbereitungsvorrichtung 4 ist zum Reinigen und/oder Desinfizieren zumindest eines medizinischen Instruments 6, insbesondere eines chirurgischen Instruments, beispielsweise eines Endoskops, eingerichtet.

Zu diesem Zweck umfasst sie einen hinter einer Tür 8 vorhandenen Aufbereitungsraum 10. Im Aufbereitungsraum 10 werden typischerweise ein oder mehrere medizinische Instrumente 6 während des Aufbereitungsvorgangs angeordnet. Die Aufbereitungsvorrichtung 4 umfasst ferner eine Bedieneinheit 12.

Ferner umfasst die Aufbereitungsvorrichtung 4 eine Steuereinheit 14, eine Drucklufteinheit 16, eine Datenbank 18, die insbesondere ein Teil der Steuereinheit 14 sein kann, sowie eine Eingabeeinheit 20 zum Erfassen eines Kennzeichnungsmerkmals des medizinischen Instruments 6. Bei dem Kennzeichnungsmerkmal handelt es sich beispielsweise um eine Seriennummer oder eine Typennummer. Insbesondere ist das Kennzeichnungsmerkmal ein maschinenlesbares Kennzeichnungsmerkmal. Beispielsweise ist als maschinenlesbares Kennzeichnungsmerkmal ein RFID-Tag, ein Barcode und/oder ein QR-Code vorgesehen. Die Eingabe des Kennzeichnungsmerkmals erfolgt beispielsweise manuell, z. B. über die Bedieneinheit 12. Ist ein maschinenlesbares Kennzeichnungsmerkmal vorgesehen, so handelt es sich bei der Eingabeeinheit 20 beispielsweise um einen RFID-Leser. Die Drucklufteinheit 16 der Aufbereitungsvorrichtung 4 umfasst einen Druckluftanschluss 22, über den die Aufbereitungsvorrichtung 4 an ein Druckluftversorgungsnetz angeschlossen wird. Während des Auslesevorgangs kann sich das maschinenlesbare Kennzeichnungsmerkmal sowohl innerhalb als auch außerhalb des Aufbereitungsraums 10 befinden.

Das medizinische Instrument 6, bei dem es sich beispielsweise um ein Endoskop handelt, weist einen innenliegenden Kanal, beispielsweise einen Arbeitskanal, auf. Nachdem das medizinische Instrument 6 verwendet wurde, muss dieser in der Figur nicht dargestellte Kanal während der Aufbereitung gereinigt bzw. gespült werden. Der innenliegende Kanal des medizinischen Instruments 6 wird mit einem Aufbereitungsfluid, beispielsweise einer Reinigungs- und Desinfektionslösung oder -lauge, gespült. Das medizinische Instrument 6 umfasst ferner ein Kennzeichnungsmerkmal 24, insbesondere ein maschinenlesbares Kennzeichnungsmerkmal, beispielsweise einen QR-Code oder ein RFID-Tag.

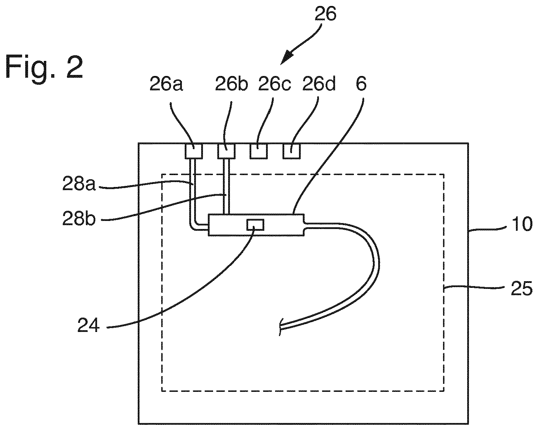

Fig. 2 zeigt den Aufbereitungsraum 10 der Aufbereitungsvorrichtung 4 in einer schematisch vereinfachten Draufsicht. Das medizinische Instrument 6 ist in einem schematisch dargestellten Aufbereitungskorb 25 angeordnet, der sich innerhalb des Aufbereitungsraums 10 befindet. In dem Aufbereitungsraum 10 sind mehrere Fluidanschlüsse 26a..26d, die allgemein auch mit Bezugszeichen 26 bezeichnet werden sollen, vorhanden bzw. angeordnet. Über die Fluidanschlüsse 26 wird das Aufbereitungsfluid dem innenliegenden Kanal des medizinischen Instruments 6 zugeführt. Beispielhaft umfasst das in Fig. 2 dargestellte medizinische Instrument 6 zwei (nicht dargestellte) innenliegende Kanäle. Entsprechend ist ein erster innenliegender Kanal über einen ersten Verbindungsschlauch 28a und der zweite Kanal über einen zweiten Verbindungsschlauch 28b mit dem ersten Fluidanschluss 26a bzw. dem zweiten Fluidanschluss 26b fluidisch gekoppelt. Die weiteren Fluidanschlüsse 26c und 26d sind nicht belegt, bleiben also frei. Allgemein gilt, dass bei dem beispielhaft dargestellten Typ des medizinischen Instruments 6 die Fluidanschlüsse 26a und 26b belegt und die Fluidanschlüsse 26c und 26d nicht belegt sind. Die Information welche Anschlüsse belegt sind und welche nicht, wird allgemein als Belegung der Fluidanschlüsse bezeichnet.

Zur Aufbereitung des medizinischen Instruments 6 wird dieses über die Verbindungsschläuche 28a, 28b mit den Fluidanschlüssen 26a, 26b fluidisch gekoppelt. Anschließend werden die innenliegenden Kanäle des medizinischen Instruments 6 mit dem Aufbereitungsfluid gespült. Wenn das medizinische Instrument 6, welches beispielsweise bereits innerhalb des Aufbereitungskorbs 25 platziert ist, in den Aufbereitungsraum 10 eingebracht wird, liest die Eingabeeinheit 20 der Aufbereitungsvorrichtung 4 das maschinenlesbare Kennzeichnungsmerkmal des medizinischen Instruments 6 aus. Alternativ wird beim Beladen der Aufbereitungsvorrichtung 4 das Kennzeichnungsmerkmal, also beispielsweise eine Seriennummer oder eine Typennummer, manuell über die Bedieneinheit 12 eingegeben. In der Datenbank 18 der Steuereinheit 14 ist eine Mehrzahl von Datensätzen gespeichert, die jeweils Informationen betreffend eine Belegung der in dem Aufbereitungsraum 10 vorhandenen Fluidanschlüsse 26 durch den betreffenden Typ eines medizinischen Instruments 6 umfasst.

Beispielsweise umfasst also die Datenbank 18 eine Vielzahl von Datensätzen, wobei jeder einzelne Datensatz einem bestimmten Typ eines Endoskops zugeordnet ist. In diesem Datensatz ist gespeichert, welcher der Fluidanschlüsse 26 durch den betreffenden Typ des Endoskops belegt wird. Im dargestellten Ausführungsbeispiel würde der zugehörige Datensatz die Information umfassen, dass das beispielhaft als medizinisches Instrument 6 dargestellte Endoskop den ersten und den ersten Fluidanschluss 26a, 26b belegt.

Diese Information wird insbesondere automatisch erfasst, wenn das medizinische Instrument 6 in den Aufbereitungsraum 10 geschoben wird, in dem nämlich das maschinenlesbare Kennzeichnungsmerkmal des medizinischen Instruments 6 von der Eingabeeinheit 20 ausgelesen wird. Die Informationen betreffend den Typ des in den Aufbereitungsraum 10 verbrachten medizinischen Instruments 6 wird von der Steuereinheit 14 mit den auf der Datenbank 18 vorhandenen Datensätzen abgeglichen. So wird nun eine Belegung der mit dem innenliegenden Kanal verbundenen Fluidanschlüsse 26a, 26b anhand des identifizierten Typs festgestellt. Die Steuereinheit 14 steuert nun die Drucklufteinheit 16 derart an, dass die belegten Fluidanschlüsse 26a, 26b mit einem Druckluftstoß beaufschlagt werden, so dass in den gespülten innenliegenden Kanälen des medizinischen Instruments 6 vorhandenes Aufbereitungsfluid aus den Kanälen ausgeblasen werden kann.

Dieser Vorgang findet beispielsweise am Ende des Aufbereitungsvorgangs statt. Die innenliegenden Kanäle des medizinischen Instruments 6 werden nicht nur von dem Aufbereitungsfluid befreit. Sie können durch einen solchen Druckluftstoß auch getrocknet werden.

Es ist ferner insbesondere vorgesehen, dass die Aufbereitung des medizinischen Instruments 6 mehrere Schritte umfasst. Beispielsweise werden die innenliegenden Kanäle des medizinischen Instruments 6 in mehreren aufeinanderfolgenden Spülschritten mit verschiedenen Aufbereitungsfluiden gespült. Zwischen zwei aufeinanderfolgenden Spülschritten werden nun die von dem medizinischen Instrument 6 belegten Fluidanschlüsse 26a, 26b mit dem Druckluftstoß beaufschlagt. Dies vermindert die Laugenverschleppung. Mit anderen Worten wird also lediglich eine unvermeidbare geringe Menge an Aufbereitungsfluid von einem Spülschritt in den nächsten übertragen.

Um sicherzugehen, dass keine Druckluft während der Beaufschlagung der Fluidanschlüsse 26 mit dem Druckluftstoß verschwendet wird, ist ferner insbesondere vorgesehen, dass die nicht belegten Fluidanschlüsse 26c, 26d zumindest während der Zeit des Druckluftstoßes abgesperrt werden. Dieses ist insbesondere für ein Druckluftsystem relevant, bei dem alle Fluidanschlüsse 26a..26d über eine gemeinsame Versorgungsleitung versorgt werden.

Gemäß einem weiteren vorteilhaften Ausführungsbeispiel ist vorgesehen, dass von dem identifizierten Typ von medizinischem Instrument 6 nicht belegte Fluidanschlüsse 26c, 26d mit einem weiteren Druckluftstoß beaufschlagt werden. Dieser weitere Druckluftstoß ist insbesondere zeitlich kürzer als der Druckluftstoß, mit dem der zumindest eine gespülte innenliegende Kanal des medizinischen Instruments 6 ausgeblasen wird. Der Vergleich der zeitlichen Dauer der beiden Druckluftstöße erfordert zur besseren Vergleichbarkeit eine Betrachtung bei gleichem Druck. Durch den weiteren Druckluftstoß wird Aufbereitungsfluid, welches sich in den nicht belegten Fluidanschlüssen 26c, 26d während des Aufbereitungsvorgangs sammelt, ausgeblasen. So ist nach Beendigung des Aufbereitungsvorgangs die Maschine optimal gereinigt und bereit für den nachfolgenden und nächsten Aufbereitungsvorgang, ohne dass Reinigungsfluid von einem in den nächsten Vorgang verschleppt wird.

Im Rahmen der Erfindung sind Merkmale, die mit "insbesondere" oder "vorzugsweise" gekennzeichnet sind, als fakultative Merkmale zu verstehen.

### Bezugszeichenliste

- 2: medizinisches System
- 4: Aufbereitungsvorrichtung
- 6: medizinisches Instrument
- 8: Tür
- 10: Aufbereitungsraum
- 12: Bedieneinheit
- 14: Steuereinheit
- 16: Drucklufteinheit
- 18: Datenbank
- 20: Eingabeeinheit
- 22: Druckluftanschluss
- 24: Kennzeichnungsmerkmal
- 25: Aufbereitungskorb
- 26, 26a..26d: Fluidanschlüsse
- 28a, 28b: Verbindungsschläuche

## Patentansprüche

1. Verfahren zum Betreiben einer Aufbereitungsvorrichtung (4) zum Reinigen und Desinfizieren zumindest eines medizinischen Instruments (6), welches zumindest einen innenliegenden Kanal aufweist, wobei das medizinische Instrument (6) während der Aufbereitung in einem Aufbereitungsraum (10) der Aufbereitungsvorrichtung (4) aufgenommen und der zumindest eine innenliegende Kanal mit einem in dem Aufbereitungsraum (10) vorhandenen Fluidanschluss (26) einer Mehrzahl vorhandener Fluidanschlüsse (26) verbunden wird, wobei der Kanal während der Aufbereitung mit einem Aufbereitungsfluid gespült wird, **dadurch gekennzeichnet, dass** die Aufbereitungsvorrichtung (4) eine Steuereinheit (14), eine Drucklufteinheit (16), eine Datenbank (18) und eine Eingabeeinheit (20) zum Erfassen eines Kennzeichnungsmerkmals (24) umfasst, wobei in der Datenbank (18) eine Mehrzahl von Datensätzen gespeichert ist, die jeweils Informationen betreffend eine Belegung der in dem Aufbereitungsraum (10) vorhandenen Fluidanschlüsse (26) durch einen bestimmten Typ eines medizinischen Instruments (6) umfassen, wobei mit der Eingabeeinheit (20) ein Kennzeichnungsmerkmal (24) des medizinischen Instruments (6) erfasst wird, auf dem Informationen betreffend seinen Typ gespeichert sind, die Steuereinheit (14) den anhand der erfassten Information identifizierten Typ des medizinischen Instruments (6) mit in der Datenbank (18) vorhandenen Datensätzen abgleicht und so eine Belegung des mit dem innenliegenden Kanal verbundenen Fluidanschlusses (26a, 26b) anhand des identifizierten Typs feststellt und die Drucklufteinheit (16) derart ansteuert, dass diese den belegten Fluidanschluss (26a, 26b) mit einem Druckluftstoß beaufschlagt, so dass in dem gespülten innenliegenden Kanal des medizinischen Instruments (6) vorhandenes Aufbereitungsfluid aus dem Kanal ausgeblasen wird, wobei die Aufbereitung des medizinischen Instruments (6) mehrere Spülschritte umfasst, **dadurch gekennzeichnet, dass** zwischen zwei aufeinanderfolgenden Spülschritten der zumindest eine belegte Fluidanschluss (26a, 26b) mit dem Druckluftstoß beaufschlagt wird.

2. Verfahren nach Anspruch 1, **dadurch gekennzeichnet, dass** von dem identifizierten Typ von medizinischem Instrument (6) nicht belegte Fluidanschlüsse (26c, 26d) zumindest während der Dauer des Druckluftstoßes abgesperrt werden, so dass aus den nicht belegten Fluidanschlüssen (26c, 26d) keine Druckluft entweicht.

3. Verfahren nach Anspruch 1 oder 2, **dadurch gekennzeichnet, dass** von dem identifizierten Typ von medizinischem Instrument (6) nicht belegte Fluidanschlüsse (26c, 26d) mit einem weiteren Druckluftstoß beaufschlagt werden, wobei dieser weitere Druckluftstoß insbesondere zeitlich kürzer ist als der Druckluftstoß mit dem der zumindest eine gespülte innenliegende Kanal des medizinischen Instruments (6) ausgeblasen wird.

4. Verfahren nach einem der Ansprüche 1 bis 3, **dadurch gekennzeichnet, dass** das Kennzeichnungsmerkmal (24) des medizinischen Instruments (6) ein maschinenlesbares Kennzeichnungsmerkmal ist und die Eingabeeinheit (20) ein Lesegerät zum Erfassen des maschinenlesbaren Kennzeichnungsmerkmals ist, wobei das Kennzeichnungsmerkmal (24) erfasst wird, indem dieses von dem Lesegerät ausgelesen wird.

5. Verfahren zum Betreiben eines medizinischen Systems (2), umfassend eine Aufbereitungsvorrichtung (4) zum Aufbereiten zumindest eines medizinischen Instruments (6) und ein medizinisches Instrument (6) mit zumindest einen innenliegenden Kanal, **dadurch gekennzeichnet, dass** das medizinische System (2) nach einem Verfahren gemäß einem der Ansprüche 1 bis 4 betrieben wird.

6. Aufbereitungsvorrichtung (4) zum Reinigen und Desinfizieren zumindest eines medizinischen Instruments (6) mit zumindest einem innenliegenden Kanal, wobei die Aufbereitungsvorrichtung (4) einen Aufbereitungsraum (10) umfasst, der zur Aufnahme des medizinischen Instruments (6) während der Aufbereitung eingerichtet ist, und wobei der zumindest eine innenliegende Kanal mit einem in dem Aufbereitungsraum (10) vorhandenen Fluidanschluss (26) einer Mehrzahl vorhandener Fluidanschlüsse (26) zum Spülen des Kanals mit einem Aufbereitungsfluid verbindbar oder verbunden ist, **dadurch gekennzeichnet, dass** die Aufbereitungsvorrichtung (4) eine Steuereinheit (14), eine Drucklufteinheit (16), eine Datenbank (18) und eine Eingabeeinheit (20) zum Erfassen eines Kennzeichnungsmerkmals (24) umfasst, wobei in der Datenbank eine Mehrzahl von Datensätzen gespeichert ist, die jeweils Informationen betreffend eine Belegung der in dem Aufbereitungsraum (10) vorhandenen Fluidanschlüsse (26) durch einen bestimmten Typ eines medizinischen Instruments (6) umfassen, und wobei die Eingabeeinheit (20) dazu eingerichtet ist, ein Kennzeichnungsmerkmal (24) des medizinischen Instruments (6) zu erfassen, in welchem Informationen betreffend den Typ des medizinischen Instruments (6) gespeichert sind, wobei die Steuereinheit (14) ferner dazu eingerichtet ist, den anhand der ausgelesenen Information identifizierten Typ des medizinischen Instruments (6) mit in der Datenbank (18) vorhandenen Datensätzen abzugleichen und so eine Belegung des mit dem innenliegenden Kanal verbundenen Fluidanschlusses (26) anhand des identifizierten Typs festzustellen und ferner die Drucklufteinheit (16) derart anzusteuern, dass diese den belegten Fluidanschluss (26a, 26b) mit einem Druckluftstoß beaufschlagt, so dass in dem gespülten innenliegenden Kanal des medizinischen Instruments (6) vorhandenes Aufbereitungsfluid aus dem Kanal ausgeblasen werden kann, wobei die Aufbereitung des medizinischen Instruments (6) mehrere Spülschritte umfasst,
**dadurch gekennzeichnet, dass** die Steuereinheit (14) ferner dazu eingerichtet ist, zwischen zwei aufeinanderfolgenden Spülschritten den zumindest einen belegten Fluidanschluss (26a, 26b) mit dem Druckluftstoß zu beaufschlagen.

7. Aufbereitungsvorrichtung (4) nach Anspruch 6, **dadurch gekennzeichnet, dass** die Steuereinheit ferner dazu eingerichtet ist, von dem identifizierten Typ von medizinischem Instrument (6) nicht belegte Fluidanschlüsse (26c, 26d) mit einem weiteren Druckluftstoß zu beaufschlagen, wobei dieser weitere Druckluftstoß insbesondere zeitlich kürzer ist als der Druckluftstoß mit dem der zumindest eine belegte Fluidanschluss (26a, 26b) beaufschlagt wird.

8. Aufbereitungsvorrichtung (4) nach Anspruch 6 oder 7, **dadurch gekennzeichnet, dass** das Kennzeichnungsmerkmal (24) des medizinischen Instruments (6) ein maschinenlesbares Kennzeichnungsmerkmal ist und die Eingabeeinheit (20) ein Lesegerät zum Erfassen des maschinenlesbaren Kennzeichnungsmerkmals ist, wobei die Steuereinheit (14) ferner dazu eingerichtet ist, das Kennzeichnungsmerkmal (24) zu erfassen, indem dieses von dem Lesegerät ausgelesen wird.

9. Medizinisches System (2), umfassend eine Aufbereitungsvorrichtung(4) nach einem der Ansprüche 6 bis 8 und zumindest ein medizinisches Instrument (6) mit zumindest einem innenliegenden Kanal, wobei das medizinische Instrument (6) ein maschinenlesbares Kennzeichnungsmerkmal umfasst, in dem Informationen betreffend den Typ des medizinischen Instruments (6) gespeichert sind.

## Claims

1. A method for operating a reconditioning apparatus (4) for cleaning and disinfecting at least one medical instrument (6) which has at least one internal channel, wherein the medical instrument (6) is received in a reconditioning chamber (10) of the reconditioning apparatus (4) during the reconditioning and the at least one internal channel is connected to a fluid connection (26) of a plurality of fluid connections (26) being present in the reconditioning chamber (10), wherein the channel is flushed during the reconditioning with a reconditioning fluid, **characterized in that** the reconditioning apparatus (4) comprises a control unit (14), a compressed air unit (16), a database (18) and an input unit (20) for detecting an identifying feature (24), wherein a plurality of data sets is stored in the database (18), which each comprise information relating to an allocation of the fluid connections (26) present in the reconditioning chamber (10) to a specific type of a medical instrument (6), wherein the identifying feature (24) of the medical instrument (6) is detected with the input unit (20), on which information relating to its type is stored, the control unit (14) compares the type of the medical instrument (6) identified on the basis of the detected information with data sets present in the database (18) and thus establishes an allocation of the fluid connection (26a, 26b) connected to the internal channel on the basis of the identified type and actuates the compressed air unit (16) **in that** the latter applies a shot of compressed air to the allocated fluid connection (26a, 26b), such that reconditioning fluid present in the flushed internal channel of the medical instrument (6) is blown out of the channel, wherein reconditioning of the medical instrument (6) comprises multiple flushing steps, **characterized in that** the shot of compressed air is applied to the at least one allocated fluid connection (26a, 26b) between two consecutive flushing steps.

2. The method according to Claim 1, **characterized in that** fluid connections (26c, 26d) which are not allocated to the identified type of medical instrument (6) are shut off at least for the duration of the shot of compressed air, such that no compressed air escapes from the unallocated fluid connections (26c, 26d).

3. The method according to Claim 1 or 2, **characterized in that** a further shot of compressed air is applied to fluid connections (26c, 26d) which are not allocated to the identified type of medical instrument (6), wherein this further shot of compressed air in particular lasts a shorter time than the shot of compressed air, with which the at least one flushed internal channel of the medical instrument (6) is blown out.

4. The method according to any one of Claims 1 to 3, **characterized in that** the identifying feature (24) of the medical instrument (6) is a machine-readable identifying feature and the input unit (20) is a reading device for detecting the machine-readable identifying feature, wherein the identifying feature (24) is detected by being read out by the reading device.

5. A method for operating a medical system (2), comprising a reconditioning apparatus (4) for reconditioning at least one medical instrument (6), and a medical instrument (6) having at least one internal channel, **characterized in that** the medical system (2) is operated in accordance to a method according to any one of Claims 1 to 4.

6. A reconditioning apparatus (4) for cleaning and disinfecting at least one medical instrument (6) having at least one internal channel, wherein the reconditioning apparatus (4) comprises a reconditioning chamber (10) which is configured to receive the medical instrument (6) during the reconditioning, and wherein the at least one internal channel can be or is connected to one fluid connection (26) of a plurality of fluid connections (26) present in the reconditioning chamber (10) in order to flush the channel with a reconditioning fluid, **characterized in that** the reconditioning apparatus (4) comprises a control unit (14), a compressed air unit (16), a database (18) and an input unit (20) for detecting an identifying feature (24), wherein a plurality of data sets is stored in the database, which each comprise information relating to an allocation of the fluid connections (26) present in the reconditioning chamber (10) to a specific type of a medical instrument (6), and wherein the input unit (20) is configured to detect the identifying feature (24) of the medical instrument (6), in which information relating to the type of the medical instrument (6) is stored, wherein the control unit (14) is further configured to compare the type of the medical instrument (6) identified on the basis of the read-out information with data sets present in the database (18) and thus establish an allocation of the fluid connection (26) connected to the internal channel on the basis of the identified type and, in addition, to actuate the compressed air unit (16) such that the latter applies a shot of compressed air to the allocated fluid connection (26a, 26b), such that reconditioning fluid present in the flushed internal channel of the medical instrument (6) can be blown out of the channel, wherein reconditioning of the medical instrument (6) comprises multiple flushing steps, **characterized in that** the control unit (14) is further configured **in that** the shot of compressed air is applied to the at least one allocated fluid connection (26a, 26b) between two consecutive flushing steps.

7. The reconditioning apparatus (4) according to Claim 6, **characterized in that** the control unit is further configured to apply a further shot of compressed air to fluid connections (26c, 26d) which are not allocated to the identified type of medical instrument (6), wherein this further shot of compressed air in particular lasts a shorter time than the shot of compressed air which is applied to the at least one allocated fluid connection (26a, 26b).

8. The reconditioning apparatus (4) according to Claim 6 or 7, **characterized in that** the identifying feature (24) of the medical instrument (6) is a machine-readable identifying feature and the input unit (20) is a reading device for detecting the machine-readable identifying feature, wherein the control unit (14) is further configured to detect the identifying feature (24) **in that** it is read out by the reading device.

9. A medical system (2), comprising a reconditioning apparatus (4) according to any one of Claims 6 to 8, and at least one medical instrument (6) having at least one internal channel, wherein the medical instrument (6) comprises a machine-readable identifying feature, in which information relating to the type of the medical instrument (6) is stored.

## Revendications

1. Procédé pour faire fonctionner un dispositif de traitement (4) destiné à nettoyer et désinfecter au moins un instrument médical (6) qui comprend au moins un canal interne, l'instrument médical (6) étant placé dans une chambre de traitement (10) du dispositif de traitement (4) pendant le traitement, et ledit au moins un canal interne est raccordé à un raccord de fluide (26) prévu dans la chambre de traitement (10) parmi une pluralité de raccords de fluide (26) prévus, le canal étant rincé avec un fluide de traitement pendant le traitement, **caractérisé en ce que** le dispositif de traitement (4) comprend une unité de commande (14), une unité à air comprimé (16), une base de données (18) et une unité de saisie (20) pour enregistrer une caractéristique d'identification (24), une pluralité d'enregistrements étant mémorisés dans la base de données (18), qui contiennent chacun des informations concernant l'occupation des raccords de fluide (26) prévus dans la chambre de traitement (10) par un type déterminé d'instrument médical (6), une caractéristique d'identification (24) de l'instrument médical (6), comprenant des informations concernant son type, étant enregistrée au moyen de l'unité de saisie (20), l'unité de commande (14) faisant correspondre le type de l'instrument médical, identifié au moyen des informations enregistrées (6), aux enregistrements présents dans la base de données (18) et déterminant ainsi une occupation du raccord de fluide (26a, 26b) relié au canal interne au moyen du type identifié, et commandant l'unité à air comprimé (16) de telle sorte que celle-ci alimente le raccord de fluide occupé (26a, 26b) avec un jet d'air comprimé, de sorte que le fluide de traitement prévu dans le canal interne rincé de l'instrument médical (6) soit expulsé du canal, le traitement de l'instrument médical (6) comprenant plusieurs étapes de rinçage,
**caractérisé en ce que**
entre deux étapes de rinçage successives, ledit au moins un raccord de fluide occupé (26a, 26b) est alimenté avec le jet d'air comprimé.

2. Procédé selon la revendication 1, **caractérisé en ce que** les raccords de fluide non occupés (26c, 26d) par le type identifié d'instrument médical (6) sont obturés au moins pendant la durée du jet d'air comprimé, de sorte qu'aucun air comprimé ne s'échappe des raccords de fluide non occupés (26c, 26d).

3. Procédé selon la revendication 1 ou la revendication 2, **caractérisé en ce que** les raccords de fluide non utilisés (26c, 26d) par le type identifié d'instrument médical (6) sont alimentés avec un autre jet d'air comprimé, cet autre jet d'air comprimé étant notamment plus court en durée que le jet d'air comprimé avec lequel ledit au moins un canal interne rincé de l'instrument médical (6) est purgé.

4. Procédé selon l'une des revendications 1 à 3, **caractérisé en ce que** la caractéristique d'identification (24) de l'instrument médical (6) est une caractéristique d'identification lisible par machine et l'unité de saisie (20) est un lecteur destiné à détecter la caractéristique d'identification lisible par machine, la caractéristique d'identification (24) étant détectée lorsqu'elle est lue par le lecteur.

5. Procédé de fonctionnement d'un système médical (2) comprenant un dispositif de traitement (4) pour traiter au moins un instrument médical (6), et un instrument médical (6) comprenant au moins un canal interne, **caractérisé en ce que** le système médical (2) est utilisé selon un procédé selon l'une des revendications 1 à 4.

6. Dispositif de traitement (4) pour nettoyer et désinfecter au moins un instrument médical (6) comprenant au moins un canal interne, le dispositif de traitement (4) comprenant une chambre de traitement (10) qui est conçue pour recevoir l'instrument médical (6) pendant le traitement, et ledit au moins un canal interne étant apte à être raccordé ou étant prévu pour être raccordé à un raccord de fluide (26) d'une pluralité de raccords de fluide (26) prévus dans la chambre de traitement (10) afin de rincer le canal avec un fluide de traitement, **caractérisé en ce que** le dispositif de traitement (4) comprend une unité de commande (14), une unité d'air comprimé (16), une base de données (18) et une unité de saisie (20) pour enregistrer une caractéristique d'identification (24), une pluralité d'enregistrements de données étant mémorisés dans la base de données, qui contiennent chacun des informations concernant l'occupation des raccords de fluide (26) prévus dans la chambre de traitement (10) par un type déterminé d'instrument médical (6), et l'unité de saisie (20) étant conçue pour détecter une caractéristique d'identification (24) de l'instrument médical (6), dans laquelle sont enregistrées des informations concernant le type de l'instrument médical (6), l'unité de commande (14) étant en outre conçue pour faire correspondre le type de l'instrument médical, identifié au moyen des informations lues (6), aux enregistrements présents dans la base de données (18), et ainsi déterminer une occupation du raccord de fluide (26) relié au canal interne au moyen du type identifié, et en outre pour commander l'unité à air comprimé (16) de telle sorte que celle-ci alimente le raccord de fluide occupé (26a, 26b) avec un jet d'air comprimé, de sorte que le fluide de traitement prévu dans le canal interne rincé de l'instrument médical (6) puisse être expulsé du canal, le traitement de l'instrument médical (6) comprenant plusieurs étapes de rinçage,
**caractérisé en ce que**
l'unité de commande (14) est en outre conçue pour alimenter le ou les raccords de fluide occupés (26a, 26b) avec le jet d'air comprimé entre deux étapes de rinçage consécutives.

7. Dispositif de traitement (4) selon la revendication 6, **caractérisé en ce que** l'unité de commande est en outre conçue pour alimenter un autre jet d'air comprimé aux raccords de fluide non occupés (26c, 26d) par le type identifié d'instrument médical (6), cet autre jet d'air comprimé étant notamment plus court en durée que le jet d'air comprimé alimenté audit au moins un raccord de fluide occupé (26a, 26b).

8. Dispositif de traitement (4) selon la revendication 6 ou la revendication 7, **caractérisé en ce que** la caractéristique d'identification (24) de l'instrument médical (6) est une caractéristique d'identification lisible par machine, et l'unité de saisie (20) est un lecteur destiné à détecter la caractéristique d'identification lisible par machine, l'unité de commande (14) étant en outre conçue pour détecter la caractéristique d'identification (24) en la lisant au moyen du lecteur.

9. Système médical (2) comprenant un dispositif de traitement (4) selon l'une des revendications 6 à 8 et au moins un instrument médical (6) avec au moins un canal interne, l'instrument médical (6) comprenant une caractéristique d'identification lisible par machine dans laquelle sont mémorisées des informations concernant le type de l'instrument médical (6).
